# EUROPEAN PATENT SPECIFICATION

(11) **EP 4 028 065 B1**
(45) Date of publication and mention of the grant of the patent: **17.07.2024**
(21) Application number: 21807019.1
(22) Date of filing: 04.11.2021
(51) Int. Cl.: A61L 2/14, A61B 1/12, A61B 90/70

(54) **APPARATUS FOR STERILISING A CHANNEL OF A SURGICAL SCOPING DEVICE**
VORRICHTUNG ZUR STERILISIERUNG EINES KANALS EINER CHIRURGISCHEN SKOPIEVORRICHTUNG
APPAREIL DE STÉRILISATION D'UN CANAL D'UN DISPOSITIF D'ENDOSCOPIE CHIRURGICALE

(30) Priority: 20.11.2020 GB 202018275
(43) Date of publication of application: 20.07.2022
(73) Proprietor: Creo Medical Limited, Chepstow, Wales NP16 5UH (GB)
(72) Inventor: HANCOCK, Christopher Paul, Chepstow, South Wales NP16 5UH (GB); HODGKINS, George, Chepstow, South Wales NP16 5UH (GB); TAPLIN, William, Chepstow, South Wales NP16 5UH (GB); LAWRENCE, Richard, Chepstow, South Wales NP16 5UH (GB); ULLRICH, George Christian, Anglesey, South Wales LL59 5HA (GB); WEBB, David Edward, Gwynedd, South Wales LL57 4DB (GB)
(74) Representative: Mewburn Ellis LLP
(86) International application number: PCT/EP2021/080683
(87) International publication number: WO 2022/106215

(56) References cited:
- WO-A1-2018/005715
- WO-A1-2019/175063
- WO-A1-2020/123679
- US-A1- 2019 232 073
- US-B2- 10 434 207

## Description

### Field of the Invention

The present invention relates to sterilisation of surgical scoping devices such as endoscopes and particularly, although not exclusively, to an apparatus which can be used to sterilise or disinfect the instrument channels of such surgical scoping devices.

### Background

Bacteria are single-celled organisms that are found almost everywhere, exist in large numbers and are capable of dividing and multiplying rapidly. Most bacteria are harmless, but there are three harmful groups; namely: cocci, spirilla, and bacilla. The cocci bacteria are round cells, the spirilla bacteria are coil-shaped cells, and the bacilli bacteria are rod-shaped. The harmful bacteria cause diseases such as tetanus and typhoid.

Viruses can only live and multiply by taking over other cells, i.e. they cannot survive on their own. Viruses cause diseases such as colds, flu, mumps and AIDS. Fungal spores and tiny organisms called protozoa can cause illness.

Such micro-organisms are known to persist in the instrument channel, or other channels, of surgical scopes (such as endoscopes, gastroscopes etc.), and it is desirable to remove these organisms. Sterilisation is an act or process that destroys or eliminates all form of life, especially micro-organisms.

Known methods of sterilising the instrument channels of scopes involve the use cleaning fluids which are flushed through the channel to expel debris. A brush may also be used to scrub the interior. The scope is then disinfected in automatic washing or disinfection units, which may involve the immersion of the scope in potentially harmful chemicals such as glutaraldehyde. Finally, the scope is rinsed thoroughly with water, then alcohol, to remove traces of the disinfectant.

Such known methods are labour-intensive, and are also prone to incomplete or insufficient sterilisation of the instrument channel. The present invention has been devised in light of the above considerations.

WO 2019/175063 A relates to a sterilisation device which uses thermal or non-thermal plasma to disinfect surgical scoping devices such as endoscopes, gastroscopes, laparoscopes and the like.

WO 2020/123679 A1 discloses endoscopic instruments and energy-based disinfection systems and methods for use with these instruments.

### Summary of the Invention

According to a first aspect of the invention, there is provided a sterilisation apparatus for sterilising a channel, such as an instrument channel, of a scoping device. The sterilisation apparatus comprises a gas supply having a conduit to deliver an ionisable gas to the channel of the surgical scoping device, a sterilisation instrument, separate from the gas supply and the conduit, configured to be inserted through the channel of a surgical scoping device. The sterilisation instrument comprises an elongate probe comprising a transmission line for conveying radiofrequency (RF) electromagnetic (EM) energy and/or microwave EM energy, and a probe tip connected at the distal end of the coaxial cable for receiving the RF and/or microwave EM energy, the probe tip comprising an electrode assembly configured to produce an electric field from the received RF and/or microwave frequency EM energy to generate a plasma of the ionisable gas delivered to the channel from the gas supply. The gases that are of interest for implementation of the apparatus disclosed herein are: air, helium, argon, nitrogen, compressed air, and carbon dioxide. The system need not be limited to these gases. Gas mixtures may be used, e.g. various concentration of argon, air and helium may be used, e.g. 1% air and 99% helium, or 5% air and 95% helium. Preferably the gas is an inert gas such as Argon or the like, though any gas which is suitable for production of a thermal or non-thermal plasma may be chosen. Preferably a gas may be selected based on the voltage which can be applied by the probe tip in order to generate a plasma. For example, air may have a higher breakdown voltage than argon, (argon has a breakdown voltage approximately 20% that of air) and so it may be preferable for the gas supply to supply argon gas in some arrangements. In particular, plasma may be created using air, but due to the high voltage required to achieve voltage breakdown other gases may be considered preferable (e.g. having a lower breakdown voltage).

In this way, the sterilisation apparatus of the present invention is arranged to flood the channel of a scoping device with an ionisable gas (i.e. substantially fill the channel with the gas, to replace the air normally present in the channel), and by producing an electric field the probe tip of the sterilisation instrument is able to strike and sustain a plasma of the gas for sterilisation and cleaning of the channel of the scoping device. The probe tip may therefore be referred to herein as a plasma applicator. The sterilisation instrument may be moved proximally and distally along the channel of the scoping device as it produces a thermal or non-thermal plasma, thereby cleaning and sterilising the channel. The thermal or non-thermal plasma may be used to provide a reduction in bioburden for a range of bacteria, including methicillin-resistant staphylococcus aureus (MRSA), clostridium difficile (c. diff.; both spores and vegetative state) and escherichia coli (e. coli), and so may allow for more efficient and thorough sterilisation of the instrument channel. The instrument may also be configured to produce a combination of non-thermal plasma and non-ionising microwave radiation.

By supplying gas to the channel of the scoping device through a separate conduit there is no need for a conduit or lumen to be formed in the sterilisation instrument itself. The sterilisation instrument may therefore have a narrower diameter than in known arrangements. For example the sterilisation instrument may have a diameter of 1 mm or less, such as 0.6mm, which may be suitable for sterilisation and cleaning of channels having a narrow diameter (e.g. around 1 mm). In contrast, known devices are not suitable for sterilising such narrow channels as the sterilisation device itself is required to convey gas therethrough for producing a plasma at the distal end. Due to the size of the conduit for delivering gas, known instruments therefore have a relatively large lower limit on their diameter, for example they may be greater than 2 mm in diameter. The present invention thereby allows small diameter channels to be cleaned and sterilised using a thermal or non-thermal plasma. Delivering gas separately of the sterilisation instrument may also provide other advantages. For example, some scoping device disinfection protocols require the scoping device to be rinsed with water, or other liquid cleaning products. By delivering a flow of gas which is separate from the sterilisation instrument, the flow of gas may also aid dispersal of water or liquid molecules which may help to dry the channel of the scoping device.

In this way, the first aspect of the invention provides the ability to perform sterilisation at the distal end of an instrument, in particular for the purpose of disinfecting the instrument channel of surgical scoping device, such as an endoscope, gastroscope, bronchoscope or the like. The apparatus allows the instrument channel to be thoroughly sterilised using a plasma which is struck and sustained by RF and/or microwave frequency EM energy, which is supplied to the probe tip from a generator.

The term "surgical scoping device" may be used herein to mean any surgical device provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube. The instrument channel may have a diameter suitable for receiving invasive surgical tools (though endoscopic procedures are usually referred to as "minimally invasive"). The diameter of the instrument channel may be 5 mm or less.

In this specification "microwave frequency" may be used broadly to indicate a frequency range of 400 MHz to 100 GHz, but preferably the range 1 GHz to 60 GHz. Specific frequencies that have been considered are: 915 MHz, 2.45 GHz, 3.3 GHz, 5.8 GHz, 10 GHz, 14.5 GHz and 24 GHz. In contrast, this specification uses "radiofrequency" or "RF" to indicate a frequency range that is at least three orders of magnitude lower, e.g. up to 300 MHz, preferably 10kHz to 1 MHz. The microwave frequency may be adjusted to enable the microwave energy delivered to be optimised. For example, a probe tip may be designed to operate at a certain frequency (e.g. 900 MHz), but in use the most efficient frequency may be different (e.g. 866 MHz). Whilst a prove tip may be designed to operate at a specific frequency, most tips will operate acceptably over a slightly wider band, where they have a non-optimal but still usable efficiency.

The elongate probe may be dimensioned to be insertable through a scoping device, e.g. through the instrument channel of an endoscope, gastroscope, bronchoscope, colonoscope or the like. For example, the coaxial cable may have a diameter of 2.5 mm or less, preferably 2.2 mm or less, or 1 mm or less. The coaxial cable may have a sleeve, wherein the sleeve may have an outer diameter less than 2.6 mm, preferably less than 2.5 mm, or less than 1.5 mm. For larger laparoscopic instruments, the outer diameter may be 3 mm or more, and larger diameter co-axial cable may be used. The coaxial cable may have a length of around 2 m or more to ensure that the probe can extend through the entire length of the instrument channel. For example, in colonoscopes, the instrument channel may be around 1.8 m in length.

Preferably, the sterilisation apparatus further comprises a gas-tight adapter configured to be connectable to the scoping device, the gas-tight adapter having a passage therethrough to allow the sterilisation instrument to be introduced into the channel of the scoping device through the gas-tight adapter. For example, the adapter may be configured to fit with a proximal end of the scoping device, and may provide access to the instrument channel of the scoping device. By being gas-tight, the adapter helps to ensure that ionisable gas provided to the channel does not leak from the channel, ensuring that sufficient gas is available for production of a plasma. The adapter also minimises ingress of air into the channel. In this way, the atmosphere within the channel may be composed entirely or substantially entirely of ionisable gas from the gas supply, wherein the gas is a suitable gas for generation of a plasma at the probe tip. This may ensure that an operator has full control of the amount of gas which is present in the channel of the scoping device to ensure optimal operation of the apparatus. In some arrangements, the gas supply (e.g. the conduit of the gas supply) is configured to be connectable to the adapter in order to deliver the ionisable gas to the channel of the surgical scoping device. For example, the gas-tight adapter may comprise a gas conduit through which gas can be delivered from the gas supply to the channel of the surgical scoping device. This provides a sterilisation apparatus which is easy to set up and operate, as it simply requires connection of the gas-tight adapter to the scoping device. In some arrangements, the gas-tight adapter may be connected to a proximal end of the scoping device and the gas supply may be connected to a distal end of the scoping device, or vice versa, such that the sterilisation instrument is introduced into the channel at a different point from the gas. This may be advantageous in ensuring a suitable amount of gas is available at the probe tip for generating a plasma.

Advantageously, the gas-tight adapter may comprise a pressure relief valve. This may allow venting of gas or air from the channel through the adapter when a predetermined pressure is reached within the channel. The pressure relief valve may be configured to ensure that gas and air can escape the channel via the valve, but no gas or air can enter the channel through the valve. For example, the predetermined pressure may ensure that, when gas is supplied to the channel of the scoping device, there is a suitable amount of gas, and/or a suitable ratio of gas to air, present in the channel to allow a thermal or non-thermal plasma to be struck and sustained by the probe tip. In some examples, gas may be continuously delivered to the channel and, by providing an escape point for excess gas, the pressure relief valve ensures that there is a flow of gas through the channel. This may be advantageous in ensuring that gas is continuously passing over the probe tip in order to produce a thermal or non-thermal plasma for sterilisation. A flow of gas may also be useful in aiding drying of the channel of the scoping device, as described above.

Preferably, the passage through the gas-tight adapter comprises a seal, such as an O-ring, a flanged seal, or the like, to ensure the adapter is gas-tight when the sterilisation instrument is positioned in the passage. Such a seal may help ensure that the adapter is gas-tight while allowing the sterilisation instrument to be moved in proximal and distal directions, thereby allowing movement the probe tip along the length of the channel for sterilisation.

Advantageously, the gas-tight adapter may comprise a luer lock fitting for fixing the gas-tight adapter to the surgical scoping device. The fitting may be suitable for attaching the adapter to a handle of a scoping device. Such a fitting enables the adapter to be fitted to a range of scoping devices, and the fitting is a gas-tight fitting. In particular, such a fitting may be configured to fit to the proximal end of a scoping device.

Preferably, the sterilisation apparatus further comprises a closure device, optimally a gas-tight closure device, configured to seal a distal end of the channel of the scoping device. For example, the closure device may comprise a cap configured to fit over a distal end of the scoping device, or may be a component which is configured to fit within the distal end of the channel. In this way, the closure device closes the distal end of the channel to ensure that air cannot enter the channel and dilute the ionisable gas atmosphere when the apparatus is in use. As with the gas-tight adapter described above, the closure device may thereby help to ensure that an operator has full control over the amount of ionisable gas which is present in the channel of the scoping device, ensuring optimal operation of the apparatus. In some examples the gas supply (e.g. the conduit of the gas supply) may be connectable to the closure device to deliver the ionisable gas to the channel of the surgical scoping device. For example, the closure device may comprise a gas conduit through which gas can be delivered from the gas supply to the channel of the surgical scoping device. In some arrangements, for example where the sterilisation instrument is introduced to the channel at its proximal end, providing connector for delivering gas to the distal end of the channel may help to ensure that a suitable amount of gas is available at the probe tip for generation of a thermal or non-thermal plasma for sterilisation of the channel. For example, such an arrangement may be particularly advantageous where the diameter of the channel is the same as, or only slightly larger than, the diameter of the sterilisation instrument, wherein the sterilisation instrument substantially occludes the channel such that insufficient ionisable gas to sustain a plasma may pass around the outside of the sterilisation instrument itself. In some embodiments, the closure device may further comprise a pressure relief valve. This may allow venting of gas or air from the channel through the closure device when a predetermined pressure is reached within the channel. The pressure relief valve may be configured to ensure that gas and air can escape the channel via the valve, but no gas or air can enter the channel through the valve. For example, the predetermined pressure may ensure that, when gas is supplied to the channel of the scoping device, there is a suitable amount of ionisable gas, and/or a suitable ratio of ionisable gas to air, present in the channel to allow a thermal or non-thermal plasma to be struck and sustained by the probe tip. In some examples, gas may be continuously delivered to the channel and, by providing an escape point for excess gas, the pressure relief valve ensures that there is a flow of gas through the channel. This may be advantageous in ensuring that gas is continuously passing over the probe tip in order to produce a thermal or non-thermal plasma for sterilisation, and may also help to dry the channel of the scoping device. In some arrangements, the closure device may have a passage therethrough to allow the sterilisation instrument to be introduced into the channel of the scoping device at the distal end. In this embodiment, the closure device may also include a seal (e.g. O-ring or flanged seal) to ensure that gas does not escape through the closure device as the instrument is advanced and retracted through the channel. For example, it is envisaged in some arrangements that ionisable gas may be introduced to the channel at its proximal end (e.g. through the gas-tight adapter) and the sterilisation instrument may be introduced to the channel at its distal end.

Preferably, the electrode assembly may comprise a first electrode and a second electrode arranged to define a volume therebetween, and the first electrode and the second electrode are configured to receive the RF and/or microwave energy from the transmission line to set up an electric field in the volume for producing a plasma of the ionisable gas. The probe tip may comprise an inlet, or at least one inlet, to allow ionisable gas to flow from the channel of the surgical scoping device and into the volume defined between the first electrode and the second electrode. By providing an inlet to a volume between the first and second electrodes in this way, the probe tip is advantageously configured to generate a plasma from ionisable gas which is present in the channel of the scoping device, as the gas is not delivered directly to the volume between the electrodes. The volume, as referred to herein, may be a space or cavity defined between the first electrode and second electrode which allows ionisable gas to be present between the electrodes to be struck to form a plasma.

The plasma may be struck using RF or microwave energy, which may be received as a high voltage pulse. Microwave energy may be used to sustain the plasma after it is struck, i.e. deliver power into the plasma to maintain the state of ionisation. This may also be received as a pulse. This arrangement may prevent electric field collapse due to the capacitance of the cable and loading variations, e.g. due to changing from a dry to a wet environment at the probe tip. Striking the plasma for delivery out of the probe tip using microwave frequency energy may be possible, e.g. by using a microwave resonator or an impedance transformer, i.e. a quarter wave transformer that transforms a low voltage to a higher voltage to strike plasma using a higher impedance transmission line that is a quarter wave (or an odd multiple thereof) long at the frequency of operation. This high impedance line may be switched in to strike plasma and switched out (i.e. to return to a lower impedance line) once the plasma has been struck and it is required to sustain plasma. A power PIN or varactor diode may be preferably used to switch between the two states, although it may be possible to use a coaxial or waveguide switch. The high electric field for striking the plasma may be caused by creating a high impedance condition for either the RF EM energy or the microwave EM energy at the probe tip.

To strike plasma it is desirable to have a high electric field (e.g. high voltage condition). In the plasma strike state (i.e. before the plasma exists) the ionisable gas is non-conducting and therefore has high impedance. In order to strike plasma, it is necessary to set-up the high impedance state at the distal end of the probe tip or within the probe tip in order to enable the high voltage (high electric field) necessary to break down the gas to be generated. The apparatus of the invention may permit the magnitude of microwave power delivered to the plasma to be controlled, e.g. through modulation of the microwave signal and control of amplifier gain or control of the level of input signal to an amplifier with fixed gain, as well as the efficiency by which it is delivered, e.g. through dynamic impedance matching. This arrangement may also enable the dosage of plasma energy delivered into the surface to be sterilised to be accurately quantified.

The impedance of the plasma is preferably matched to the impedance of the probe tip (and energy delivery system) at the frequency of the microwave energy to enable efficient transfer of the microwave energy, produced by the generator, into the plasma. Where microwave energy is used, the probe tip and/or generator may be tuned (statically or dynamically) to ensure that the plasma is matched into the load presented by the instrument channel and material within the channel. At microwave frequencies, the coaxial cable forms a distributed element transmission line, where the impedance match between the probe tip and energy source is determined by the source impedance of the microwave generator, the characteristic impedance of the coaxial cable (transmission line), and the impedance of the probe tip structure itself. If the characteristic impedance of the coaxial cable is the same as the output impedance of the source then all of the microwave power will be delivered into the probe tip, less the attenuation caused by the coaxial cable (dielectric and conductor losses). If the impedance of the probe tip and the instrument channel is the same as the characteristic impedance of the coaxial cable, then the maximum power available at the source will be transferred into the plasma/instrument channel load. Adjustments may be made to probe tip structure in order to maintain the best impedance match between the probe tip and the plasma/instrument channel load. Adjustments may also be made at the generator or at the interface between the distal end of the first cable and the proximal end of the second (instrument) cable. These adjustments may be in the form of a change of capacitance and/or inductance of a matching network, i.e. stub tuning.

The apparatus may use, as a generator, a source oscillator to produce a low power microwave frequency signal and a power amplifier (e.g. an arrangement of microwave transistors) to amplify the low power signal to a level that is high enough to enable an electric field to be produced which is required to strike the plasma using a gas found to be suitable for the particular application. Solid state signal amplifiers may be used. The system may also operate in a mode whereby the amplifier is driven into saturation or full power to set up an electric field necessary to strike the plasma and then backed off once it has been struck. The ability to control the microwave energy can enable a plasma to be generated that is most suitable for any one of a variety of applications of interest. Control of the microwave energy and/or the gas flow rate and/or the gas mixture gives control over the size of the plume and the temperature at the inner surface of the instrument channel being treated. Furthermore, the system may be arranged to quantify the dosage of plasma energy delivered to the surface to be treated. The microwave energy may be controlled by any one or more of varying a frequency of the microwave energy in a controlled manner (e.g. controlling the frequency of radiation from the microwave radiation generator), varying the power level in a controlled manner, and modulating the microwave energy in a controlled manner. The generator may include a microwave signal modulator arranged to modulate the microwave energy delivered to the probe tip. The modulation frequency may be contained within the range from 0.1 Hz up to 10 MHz. The duty cycle may be from less than 1% to 100%. In some embodiments, the modulation frequency may be from 10 Hz to 100 kHz and the duty cycle may be between 10% and 25%. In preferred embodiments the modulation frequency may be between 100 Hz and 1 kHz, and the duty cycle may be 20%.

The apparatus may thus be arranged to generate the plasma using pulsed operation. In one embodiment, the plasma may be struck on each pulse (the strike may occur due to a transient produced on one of the edges of the pulse - normally the positive going edge). The operation of the system may be such that it is necessary to keep applying pulses to the system in order to generate the required effects.

Preferably, the transmission line comprises a coaxial cable having an inner conductor, an outer conductor and a dielectric material separating the inner conductor from the outer conductor. The probe tip may comprise the second electrode, comprising an extension of the second conductor of the coaxial cable beyond the dielectric material of the coaxial cable, and the first electrode, comprising an extension of the inner conductor of the coaxial cable beyond the dielectric material of the coaxial cable and along a central or longitudinal axis of the second electrode to define the volume between the first electrode and the second electrode; wherein the first electrode and the second electrode are configured to receive the RF and/or microwave energy from the coaxial cable to set up an electric field in the volume between the first electrode and the second electrode for striking a plasma therein, and wherein the probe tip includes an outlet for releasing plasma from the volume. For example, the distal end face of the probe tip may be open to define the inlet to allow ionisable gas to flow from the channel of the surgical scoping device and into the internal volume. In some examples, a portion of the opening may provide an inlet for gas from the channel to flow into the internal volume and a portion of the opening may provide an outlet for plasma to escape the internal volume, wherein a flow may be generated within the internal volume due to the generation of plasma. In this way, the probe tip is adapted for use in an environment filled with a gas suitable for generating a thermal or non-thermal plasma rather than having such gas delivered directly to the region between the electrodes. The relatively simple design of the probe tip is also simple and cheap to manufacture, and is suitable for use even when the probe tip has a small diameter. Optionally, the second electrode may comprise at least one aperture to define the inlet to allow ionisable gas to flow from the channel of the surgical scoping device into the space or volume defined between the first electrode and the second electrode. The aperture or apertures may thereby help ensure that a thermal or non-thermal plasma is continuously produced in use. The aperture or apertures may also allow plasma out of the region between the first electrode and the second electrode, and the plasma may be directed at a sidewall of the channel for sterilisation.

Advantageously, the first electrode may further comprise a conductive cap mounted on a distal end of the extension of the inner conductor, wherein the conductive cap is spaced away from the distal end of the second electrode to define a gap between the cap and the second electrode to define the outlet. By being arranged in this way, plasma produced by the probe tip may be preferentially directed at a sidewall of the channel to ensure thorough sterilisation and cleaning. In some examples, the conductive cap may comprise at least one aperture to define the inlet to allow gas to flow into the space or volume defined between the first electrode and the second electrode. The aperture or apertures may thereby help ensure that a thermal or non-thermal plasma is continuously produced in use. The aperture or apertures may also allow plasma out of the region between the first electrode and the second electrode. In some examples, the cap may instead be made of a ceramic material.

As explained above, the high electric field for striking the plasma may be caused by creating a high impedance condition for either the RF EM energy or the microwave EM energy at the probe tip. This can be achieved through the selection of a suitable geometry for the first and second electrodes. For example, a piece of insulating dielectric material, such as quartz or other similarly low loss material, may be located between the first and second electrodes. This may increase the impedance and therefore facilitate the creation of a high electric field.

Preferably, the gas supply may be configured to supply gas to the channel of the surgical scoping device at a variable flow rate. For example, the sterilisation apparatus may include a flow controller arranged to adjustably control gas flow. In particular, the flow rate of gas may be between 0.2 and 10 litres per minute, and may be varied between those values. By varying the flow rate of gas delivered to the channel it can be ensured that there is a suitable supply of gas available at the probe tip. For example, as the channel may be restricted by the presence of the sterilisation instrument, a higher gas flow rate may be needed to deliver gas from a proximal end of the channel to the probe tip when the probe tip is at the distal end of the channel when compared with a flow rate of gas required when the probe tip is closer to the proximal end of the channel. The flow rate of gas may also be varied in order to provide or control drying of the channel if liquids (e.g. cleaning liquids) are present in the channel, in small quantities.

Herein, the term "inner" means radially closer to the centre (e.g. axis) of the channel and/or coaxial cable. The term "outer" means radially further from the centre (axis) of the channel and/or coaxial cable. The term "conductive" is used herein to mean electrically conductive, unless the context dictates otherwise. Herein, the terms "proximal" and "distal" refer to the ends of the elongate probe. In use the proximal end is closer to a generator for providing the RF and/or microwave energy, whereas the distal end is further from the generator.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1** shows a schematic diagram of a sterilisation apparatus according to an embodiment of the present invention, and a generator for supplying energy thereto;
**Figure 2** shows a schematic diagram of a sterilisation apparatus according to a second embodiment of the present invention in use;
**Figure 3** shows a schematic diagram of a sterilisation apparatus according to a third embodiment of the present invention in use;
**Figure 4** shows a cross-section view of a first probe tip which may be used with embodiments of the present invention;
**Figure 5** shows a cross-section view of a second probe tip which may be used with embodiments of the present invention; and
**Figure 6** shows a cross-section view of an adapter which may be used with embodiments of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

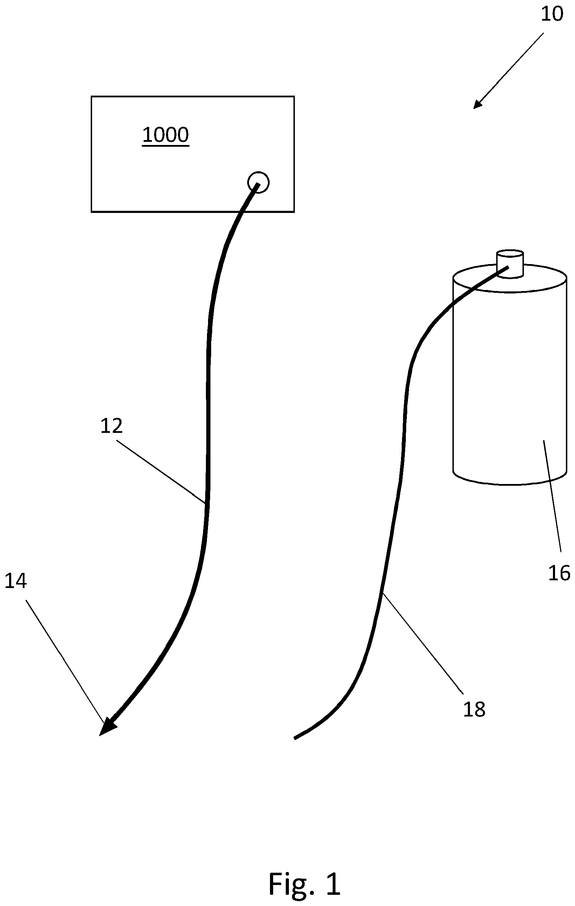

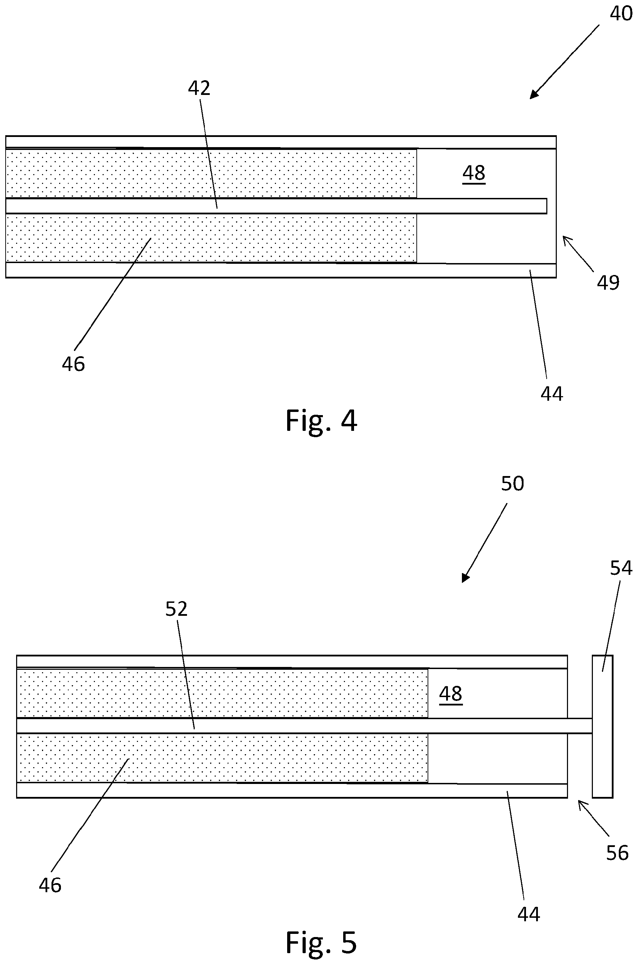

Fig. 1 shows a sterilisation apparatus 10 according to an embodiment of the present invention, and a generator 1000 for supplying energy thereto. The sterilisation apparatus 10 comprises a sterilisation instrument having an elongate probe comprising a transmission line provided by a coaxial cable 12 with a probe tip 14 at its distal end. For example, the probe tip 14 may be a probe tip as described below with respected to Figs. 4 or 5. The generator 1000 is connected to the coaxial cable 12 at its proximal end, and is configured to supply radiofrequency (RF) and/or microwave frequency electromagnetic (EM) energy in order to facilitate the production of plasma by an electrode assembly at the probe tip 14. The apparatus 10 also comprises a gas supply 16 having a gas conduit 18, separate from the sterilisation instrument, to deliver an ionisable gas to the channel of a surgical scoping device, in a manner which will be described in more detail below. The gas supply 16 may be a canister of gas, as shown, but any suitable gas supply may be used. Preferably the gas supply 16 provides an inert gas, such as Argon. Although not shown in Fig. 1, in some examples the gas supply 16 may comprise a gas flow controller allowing the flow rate of gas to be varied. For example, the gas flow rate may be between 0.2 and 10 litres per minute, and may be varied automatically or manually by a user. For example, the flow rate may be increased when the probe tip is towards the distal end of the channel in a scoping device in order to ensure that a suitable supply of gas is available for production of a plasma.

During a sterilisation process, with the probe tip 14 positioned within a channel of a scoping device, the generator 1000 supplies RF and/or microwave frequency EM energy to the probe tip. The gas supply 16 simultaneously supplies gas directly to the channel via the gas conduit 18. The RF and/or microwave energy and supplied gas are combined at the probe tip 14 to generate a thermal or non-thermal plasma, which is emitted from the probe tip 14 to contact a surface of the channel to destroy or eliminate micro-organisms. Examples of plasma generation in a similar manner are disclosed in WO 2009/060213 A1, for example.

The generator 1000 may be controlled to determine whether the generated plasma is a non-thermal or thermal plasma. For example, the supply microwave energy may have a power and/or duty cycle that is selectable to produce non-thermal or thermal plasma. Preferably, the generator is operated to produce a non-thermal plasma having a temperature of less than 41 °C, which can help avoid damage to the scoping device.

The apparatus 10 may further include a withdrawal device (not shown) coupled to the coaxial cable 12 and operable to withdraw the coaxial cable 12 through the channel at a predetermined rate.

In use, the sterilisation instrument is inserted through the channel of a surgical scoping device, and the gas conduit is arranged to deliver gas directly to the channel of the scoping device. For example, both the sterilisation instrument and the gas conduit may be provided to a proximal end of the channel or to a distal end of the channel. Alternatively, the sterilisation instrument and the gas conduit may be provided to opposing ends of the channel. Comparative advantages of these arrangements are described herein.

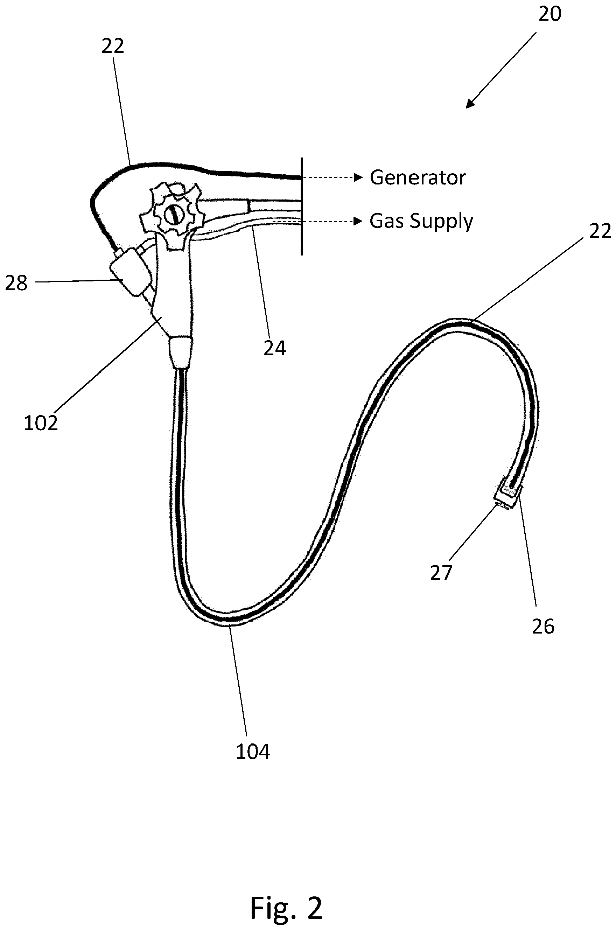

Fig. 2 shows a sterilisation apparatus 20 according to a further embodiment of the invention, the apparatus 20 being shown in use for cleaning a surgical scoping device. The elongate probe is positioned within an instrument channel of an insertion tube 104 of a surgical scoping device. The coaxial cable 22 of the elongate probe passes into the channel through the handle 102 of the scoping device, at the proximal end of the insertion tube 104 and instrument channel, and reaches the distal end of the insertion tube 104 where the electrode assembly of the probe tip (not shown) produces a plasma for sterilising and cleaning the instrument channel. For example, the probe tip may be a probe tip as described below with respected to Figs. 4 or 5. The coaxial cable 22 is connected to a generator at its proximal end (not shown), as described above with respect to Fig. 1, and conveys energy from the generator to the probe tip to enable the probe tip to generate an electric field for producing a thermal or non-thermal plasma. To sterilise and clean the channel, the elongate probe may be moved in a proximal and/or distal direction while such plasma is generated at the probe tip.

In this arrangement, a distal end of the gas conduit 24 of the apparatus 20 is also connected to a proximal end of the insertion tube 104 to deliver ionisable gas into the instrument channel. The gas conduit 24 is connected to a gas supply at its proximal end (not shown), as described above with respect to Fig. 1. The gas supply is thereby able to substantially fill the instrument channel with ionisable gas in order to provide a suitable atmosphere for the probe tip to strike and sustain a plasma. By delivering ionisable gas to the instrument channel in this way, there is no need for the elongate probe to comprise a lumen for conveying gas to the probe tip, and so the elongate probe is simpler and cheaper to manufacture, and is also more compact allowing channels of small diameters (e.g. around 1 mm or less) to also be cleaned and sterilised by the apparatus of the present invention.

In this embodiment, the apparatus 20 further comprises a gas-tight adapter 28. The adapter 28 is configured to be connected to the handle 102 of the scoping device, and in particular may be configured to fit to an input port for the instrument channel of the scoping device, and provides a passage through which the elongate probe is introduced into the channel of the scoping device. The passage comprises a seal, such as an O-ring, which ensures that the adapter is gas-tight while the sterilisation instrument is positioned in the passage, but allows the elongate probe to be moved in proximal and distal directions for cleaning and sterilisation of the channel. The distal end of the gas conduit 24 is connected to the adapter 28, and the adapter 28 has a corresponding conduit to provide ionisable gas into the channel such that the probe tip is able to produce a plasma of the gas. The adapter 28 helps to ensure that the atmosphere within the channel is suitable for production of a plasma, for example by ingress of air which could otherwise displace or dilute gas provided via the conduit 24 and thereby reduce plasma production. In order to be fixed to the handle 102 of the scoping device, the adapter 28 may comprise a luer lock fitting, though it will be appreciated that any suitable gas-tight fitting may be chosen. As the adapter 28 is configured to receive both the coaxial cable 22 and the gas conduit 24, the sterilisation apparatus 20 is particularly easy to use, as only one connection to the scoping device is required. This may be particularly advantageous when the apparatus 20 is to be transferred between multiple scoping devices for quick cleaning and sterilisation of the instrument channels.

For closing the distal end of the channel through the insertion tube 104, the sterilisation apparatus 20 comprises a closure device in the form of a cap 26 which is configured to fit over the distal end of the insertion tube 104 of the surgical scoping device. For example, the cap 26 may be generally cylindrical with an open end to fit over the insertion tube 104 and a closed end to prevent gas from escaping. The cap 26 may be made of a gas-impermeable material, such as a plastic material, for example rubber or the like, and is suitably dimensioned to ensure a tight fit with the distal end of the insertion tube 104. The cap 26 may help to prevent ingress of ambient air which could displace or dilute ionisable gas provided to the channel through the conduit 24. However, in some examples it is believed that the cap 26 may not be necessary if the flow rate of ionisable gas through the conduit 24 is high enough, as the gas flow from the distal end of the channel may be sufficient to prevent ingress of ambient air. Nevertheless, the cap 26 may be preferable, particularly where a low flow rate of gas is used. As shown in Fig. 2, the cap 26 comprises a pressure relief valve 27 which may be positioned on the closed end of the cap 26. In this way, the cap 26 is adapted to allow venting of excess gas or air from the channel through the pressure relief valve 27 when a predetermined pressure is reached. By providing the valve 27 in the cap 26, it may be ensured that there is a flow of gas through the channel when the apparatus 20 is in use, which may be particularly advantageous in ensuring that gas is continuously passing over the probe tip in order to produce a thermal or non-thermal plasma. For example, the valve 27 may be adapted to vent gas when the pressure is about or slightly above atmospheric pressure.

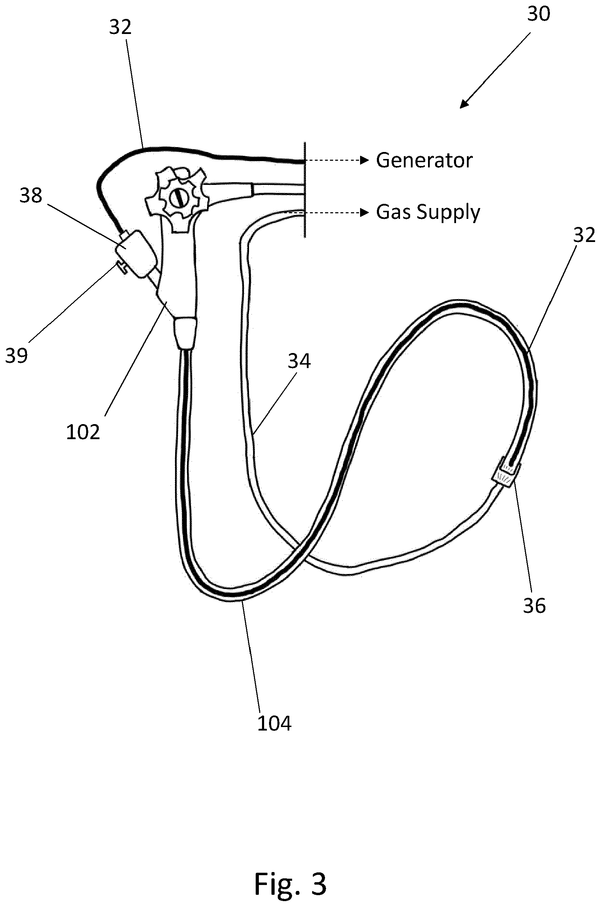

Fig. 3 shows a further embodiment of a sterilisation apparatus 30 according to an embodiment of the invention, the apparatus 30 being shown in use for cleaning a surgical scoping device. The elongate probe of the apparatus 30 is positioned within an instrument channel of an insertion tube 104 of a surgical scoping device. The coaxial cable 32 of the elongate probe passes into the channel through the handle 102 of the scoping device, at the proximal end of the insertion tube 104 and of the instrument channel, and reaches the distal end of the insertion tube 104 wherein the electrode assembly of the probe tip (not shown) produces a plasma for sterilising and cleaning the instrument channel. For example, the probe tip may be a probe tip as described below with respected to Figs. 4 or 5. The coaxial cable 32 is connected to a generator at its proximal end (not shown) as described above with respect to Fig. 1, and conveys energy from the generator to the probe tip, where an electric field is produced to generate a plume of thermal or non-thermal plasma. To sterilise and clean the channel, the elongate probe may be moved in a proximal and/or distal direction while plasma is produced at the probe tip.

In this arrangement, a distal end of the gas conduit 34 of the apparatus 30 is connected to a distal end of the insertion tube 104 to deliver gas into the instrument channel. The gas conduit 34 is connected to a gas supply at its proximal end (not shown), as described above with respect to Fig. 1. The gas conduit 34 is connected to the distal end of the insertion tube 104 by a closure device in the form of a cap 36 which is configured to fit over the distal end of the insertion tube 104 and which has a corresponding gas conduit therethrough to allow gas to enter the instrument channel. For example, the cap 36 may be generally cylindrical and may have an open end to fit over the insertion tube 104 and a connector at an opposing end for connection to the distal end of the gas conduit 34. The cap 36 may be made of a gas-impermeable material, for example a plastic material such as rubber or the like, and is suitably dimensioned to ensure a tight fit with the distal end of the insertion tube 104. As well as providing an inlet for gas from the gas conduit 34, the cap 36 may help to prevent ingress of ambient air which could displace or dilute gas provided to the channel through the conduit 34.

A gas-tight adapter 38 is provided at the proximal end of the surgical scoping device, and is configured to be connected to the handle 102. The adapter 38 may be configured to fit to an input port for the instrument channel of the scoping device, and provides a passage through which the elongate probe is introduced into the channel of the scoping device. As described above with respect to Fig. 2, this passage may comprise a seal, such as an O-ring, to ensure that the passage is gas-tight when the sterilisation instrument is positioned in the passage. The adapter 38 helps to ensure that the atmosphere within the channel is suitable for production of a plasma, for example by ingress of air which could otherwise displace or dilute gas provided via the conduit 34 and thereby reduce plasma production. In order to be fixed to the handle 102 of the scoping device, the adapter 38 may comprise a luer lock fitting, though it will be appreciated that any suitable gas-tight fitting may be chosen. In this embodiment, the gas-tight adapter 38 comprises a pressure relief valve 39, which is configured to allow venting of excess gas or ait from the channel through the pressure relief valve 39 when a predetermined pressure is reached. Preferably, the predetermined pressure may be about or slightly above atmospheric pressure.

The sterilisation apparatus 30 thereby provides for the sterilisation instrument and the gas conduit to be provided at opposing ends of the instrument channel which is to be sterilised. This may be particularly advantageous in cleaning channels which are entirely or substantially entirely occluded by the coaxial cable 32, which would inhibit a flow of gas to the probe tip if gas were to be introduced at the proximal end of the instrument channel.

Fig. 4 shows a cross-section view of a probe tip 40 which may be used with embodiments of the present invention. The probe tip 40 shown uses extension of inner and outer conductors of a coaxial cable to provide an electrode assembly having first and second electrodes, but it will be appreciated that in some examples the probe tip 40 may be provided as a separate component, or that either of the first and second electrodes may be separate from the coaxial cable.

The probe tip 40 comprises a first electrode 42 and a second electrode 44 which is coaxial with the first electrode 42. A dielectric material 46 is provided coaxially between the first electrode 42 and the second electrode 44. For example, the dielectric material 46 may be the dielectric of the coaxial cable. The second electrode 44, comprising an extension of the outer conductor of the coaxial cable, extends beyond the dielectric material 46 in a distal direction to define an internal volume 48. For example, the second electrode 44 may have a length of 5 mm or less, such as around 3 mm, beyond the distal end of the dielectric material 46. The first electrode 42, comprising an extension of the inner conductor of the coaxial cable beyond the dielectric material 46 in a distal direction, extends into the internal volume 48. For example, the first electrode 42 may run along a central, longitudinal axis of the second electrode 44, and thereby define the volume between the first electrode 42 and the second electrode 44, which may be referred to as the internal volume 48. As shown in Fig. 4, the first electrode 42 may be substantially linear, but it will be appreciated that the first electrode could also take other shapes in the internal volume 48, for example the first electrode 42 may form a helix.

The distal end of the probe tip 40 is open to define an inlet for allowing ionisable gas from the channel of the scoping device to enter the internal volume 48, where the gas may be struck to form a plasma. The opening, or a portion of the opening, at the distal end may also define an outlet for releasing plasma from the internal volume 48. For example, the second electrode 44 may be generally cylindrical and open at its distal end to define the inlet and the outlet. Gas may flow into the internal volume 48 where a plasma is struck by an electric field generated between the first electrode 42 and the second electrode 44 when RF and/or microwave EM energy is delivered thereto from the generator. The plasma which is produced is released through the outlet in order to sterilise and clean the sidewall of the instrument channel of a surgical scoping device. In some embodiments, the electric field between the first electrode 42 and the second electrode 44 may be increased by positioning a piece of insulating dielectric material, such as quartz, between the electrodes. For example, a cylindrical piece of quartz may be fitted over the first electrode 42 within the internal volume 48 to increase impedance and thus facilitate the creation of a high electric field to strike and sustain a plasma. In some examples, the second electrode 44 may comprise a number of apertures (i.e. one or more) which provide inlets enabling ionisable gas to flow into the internal volume 48. The apertures may also provide outlets through which plasma may be released from the internal volume 48. A flow of gas and plasma may be established through the internal volume 48 due to the production of plasma.

Fig. 5 shows a cross-section view of a second probe tip 50 which may be used with embodiments of the present invention. The probe tip 50 is generally similar to that shown in Fig. 4 so corresponding elements have been given corresponding reference numerals, and description thereof is not repeated.

In the probe tip 50 shown in Fig. 5, the first electrode 52 extends beyond the distal end of the second electrode 44 and a conductive cap 54 is mounted at the distal end of the first electrode 52. The conductive cap 54 may be provided as a disc of metal, such as copper, silver, gold or plated steel for example. In other embodiments, the cap 54 may be made of a ceramic material. The cap 54 is spaced away from the distal end of the second electrode 44 to define an outlet 56 through which plasma may be released from the internal volume 48. For example, the conductive cap 54 may be spaced around 0.5 mm away from the distal end of the second electrode 44. The outet 56 defined in this way is generally annular, and so plasma which is generated by the probe tip 50 is preferentially directed towards the sidewalls of the channel in the scoping device for more effective cleaning and sterilisation. In some embodiments, the conductive cap 54 may comprise a number (i.e. one or more) of apertures therethrough, which may provide inlets allowing ionisable gas to flow into the internal volume 48 from the channel in order to be struck to produce a plasma.

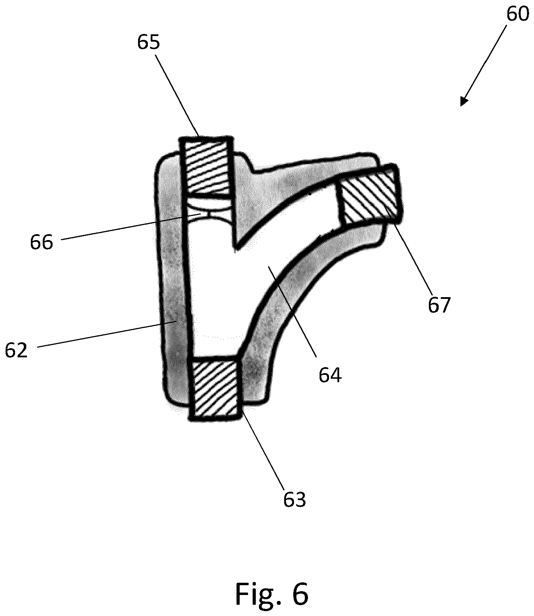

Fig. 6 shows a cross-sectional view of an adapter 60 which may be used in embodiments of the present invention. In particular, the adapter 60 may be used in an arrangement such as shown in Fig. 2 as it is configured to be connectable to a gas conduit for delivering ionisable gas to the channel of a scoping device.

The adapter 60 comprises a gas-tight body 62 which is configured to fit to the channel of a scoping device (for example, by fitting to the handle of a scoping device) to allow a sterilisation instrument to be introduced to the channel. For example, the body 62 may be made of a plastics material or the like, though any other suitable material (e.g. metal) may also be used. In the embodiment shown in Fig. 6, the adapter 60 is also configured to allow ionisable gas to be introduced to the channel of the scoping device through the gas-tight body 62.

In particular, the adapter 60 comprises a first luer lock fitting 63 to allow the gas-tight body 62 to fit to the channel of the scoping device, preferably by fitting to the handle of a scoping device as described above. Within the body 62, the adapter 60 comprises a branched passage 64 having two inlets and a single outlet, wherein the outlet is at the luer lock fitting 63 such that the outlet leads to the channel of the scoping device when the adapter 60 is in use.

At a first inlet of the passage 64 there is a port 65 which is configured to receive the elongate probe of the sterilisation device. At or about the port 65 there is a seal 66, which may be an O-ring or a flanged seal or the like, to ensure that when the elongate probe is present in the passage 64 the adapter 60 is gas-tight such that no ionisable gas may leak from the channel of the scoping device and no air may enter the channel. However, the port 65, seal 66 and the passage 64 are configured to allow the elongate probe to be moved in proximal and distal directions to sterilise the channel of the scoping device.

At a second inlet of the passage 64 there is a second luer lock fitting 67 to which a gas conduit from a gas supply may be connectable. In this way, the passage 64 may provide a conduit for passing gas from the gas supply to the channel of the scoping device. In other embodiments, the adapter 60 may not comprise a second luer lock fitting 67 (for example, where ionisable gas is delivered to the distal end of the channel of the scoping device) and the passage 64 may therefore not need to be a branched passage.

In some examples, the adapter 60 may comprise a pressure relief valve (not shown), which may be configured to vent gas when the pressure within the adapter 60 (and therefore within the channel of the scoping device) is about or slightly above atmospheric pressure). For example, the pressure relief valve may replace the second luer lock fitting 67, or may be positioned elsewhere on the body 62 of the adapter 60.

While the invention has been described in conjunction with the exemplary embodiments described above, many modifications and variations within the scope of the appended claims will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A sterilisation apparatus for sterilising a channel of a surgical scoping device, the
apparatus comprising:
a gas supply (16) ,
a sterilisation instrument configured to be inserted through the channel of a surgical scoping device, the sterilisation instrument comprising:
an elongate probe comprising:
a transmission line (32) for conveying radiofrequency (RF) electromagnetic (EM) energy and/or microwave EM energy, and
a probe tip (40) connected at the distal end of the transmission line for receiving the RF and/or microwave EM energy, the probe tip (40) comprising an electrode assembly configured to produce an electric field from the received RF and/or microwave frequency EM energy to generate a plasma of the ionisable gas delivered to the channel from the gas supply (16), **characterised in that**:
the gas supply has a conduit to deliver an ionisable gas to the channel of the surgical scoping device, and **in that**
the sterilisation instrument is separate from the gas supply and the conduit, and **in that** the sterilization instrument is free of a conduit for conveying ionisable gas for generation of a plasma by the electrode assembly.

2. A sterilisation apparatus according to claim 1, the sterilisation apparatus (10, 20) further comprising a gas-tight adapter (28, 60) configured to be connectable to the scoping device, the gas-tight adapter having a passage therethrough to allow the sterilisation instrument to be introduced into the channel of the scoping device through the gas-tight adapter (28, 60).

3. A sterilisation apparatus according to claim 2, wherein either:
the gas-tight adapter (28, 60) comprises a pressure relief valve (27); or
the gas supply (16) is configured to be connectable to the adapter (28, 60) in order to deliver the ionisable gas to the channel of the surgical scoping device.

4. A sterilisation apparatus according to claim 2 or claim 3, wherein the passage through the gas-tight adapter (28, 60) comprises a seal to ensure the adapter (28, 60) is gas-tight when the sterilisation instrument is positioned in the passage.

5. A sterilisation apparatus according to any one of claims 2 to 4, wherein the gas-tight adapter (60) comprises a luer lock fitting (63) for fixing the gas-tight adapter (28, 60) to the surgical scoping device.

6. A sterilisation apparatus according to any one of the preceding claims, the sterilisation apparatus (10, 20) further comprising a closure device (26) configured to seal a distal end of the channel of the surgical scoping device.

7. A sterilisation apparatus according to claim 6, wherein either:
the gas supply (16) is connectable to the closure device (26) to deliver the ionisable gas to the channel of the surgical scoping device; or
the closure device (26) comprises a pressure relief valve (27).

8. A sterilisation apparatus according to any one of the preceding claims, wherein electrode assembly comprises a first electrode (42) and a second electrode (44) arranged to define a volume (48) therebetween, and the first electrode (42) and the second electrode (44) are configured to receive the RF and/or microwave energy from the transmission line to set up an electric field in the volume (48) for producing a plasma of the ionisable gas; and
wherein the probe tip (40) comprises an inlet to allow ionisable gas to flow from the channel of the surgical scoping device and into the volume (48) defined between the first electrode (42) and the second electrode (44).

9. A sterilisation apparatus according to claim 8, wherein
the transmission line comprises a coaxial cable having an inner conductor, an outer conductor, and a dielectric material separating the inner conductor from the outer conductor,
the second electrode (44) comprises an extension of the outer conductor of the coaxial cable beyond the dielectric material of the coaxial cable, and
the first electrode (42) comprises an extension of the inner conductor of the coaxial cable beyond the dielectric material of the coaxial cable and along a central axis of the second electrode (44) to define the volume (48) between the first electrode (42) and the second electrode (44);
wherein the first electrode (42) and the second electrode (44) are configured to receive the RF and/or microwave energy from the coaxial cable to set up an electric field in the volume (48) between the first electrode (42) and the second electrode (44) for striking a plasma therein, and
wherein the probe tip (40) includes an outlet for releasing plasma from the volume (48).

10. A sterilisation apparatus according to claim 9, wherein the second electrode (44) comprises at least one aperture to define the inlet in the probe tip (40) to allow ionisable gas to flow from the channel of the surgical scoping device and into the volume (48).

11. A sterilisation apparatus according to claim 9 or claim 10, wherein the first electrode (42) comprises a conductive cap (54) mounted on a distal end of the extension of the inner conductor, wherein the conductive cap (54) is spaced away from the distal end of the second electrode (44) to define a gap between the cap and the second electrode (44) to define the outlet.

12. A sterilisation apparatus according to claim 11, wherein the conductive cap (54) comprises at least one aperture to define the inlet in the probe tip (40) to allow ionisable gas to flow from the channel of the surgical scoping device and into the volume (48).

13. A sterilisation apparatus according to any one of claims 9 to 12, wherein the probe tip (40) further comprises a piece of insulating dielectric material positioned between the first electrode (42) and the second electrode (44).

14. A sterilisation apparatus according to any one of the preceding claims, wherein the elongate probe has a diameter of 1mm or less.

15. A sterilisation apparatus according to any one of the preceding claims, wherein the gas supply (16) is configured to supply gas to the channel of the surgical scoping device at a variable flow rate.

## Patentansprüche

1. Sterilisationsvorrichtung zum Sterilisieren eines Kanals einer chirurgischen Sondierungsvorrichtung, wobei die Vorrichtung Folgendes umfasst:
eine Gaszufuhr (16),
ein Sterilisationsinstrument, das ausgelegt ist, um durch den Kanal einer chirurgischen Sondierungsvorrichtung eingeführt zu werden, wobei das Sterilisationsinstrument Folgendes umfasst:
eine längliche Sonde, umfassend:
eine Übertragungsleitung (32) zum Übertragen von elektromagnetischer (EM) Hochfrequenz- (HF-) Energie und/oder Mikrowellen-EM-Energie und
eine Sondenspitze (40), die mit dem distalen Ende der Übertragungsleitung zum Empfangen der HF- und/oder Mikrowellen-EM-Energie verbunden ist, wobei die Sondenspitze (40) eine Elektrodenanordnung umfasst, die ausgelegt ist, um aus der empfangenen elektromagnetischen HF- und/oder Mikrowellenfrequenz-EM-Energie ein elektrisches Feld zu erzeugen, um ein Plasma des ionisierbaren Gases, das aus der Gaszufuhr (16) in den Kanal zugeführt wurde, zu erzeugen, **dadurch gekennzeichnet, dass**:
die Gasversorgung eine Leitung aufweist, um ein ionisierbares Gas in den Kanal der chirurgischen Sondierungsvorrichtung zuzuführen, und dass das Sterilisiationsinstrument von der Gaszufuhr und der Leitung getrennt ist, und dass das Sterilisationsinstrument keine Leitung zum Transportieren eines ionisierbaren Gases zur Erzeugung eines Plasmas durch die Elektrodenanordnung aufweist.

2. Sterilisationsvorrichtung nach Anspruch 1, wobei die Sterilisationsvorrichtung (10, 20) ferner einen gasdichten Adapter (28, 60) umfasst, der ausgelegt ist, um mit der Sondierungsvorrichtung verbindbar zu sein, wobei der gasdichte Adapter einen Durchgang durch ihn hindurch aufweist, um zu ermöglichen, dass das Sterilisationsinstrument durch den gasdichten Adapter (28, 60) in den Kanal der Sondierungsvorrichtung eingeführt wird.

3. Sterilisationsvorrichtung nach Anspruch 2, wobei entweder:
der gasdichte Adapter (28, 60) ein Druckablassventil (27) umfasst; oder
die Gaszufuhr (16) ausgelegt ist, um mit dem Adapter (28, 60) verbindbar zu sein, um das ionisierbare Gas in den Kanal der chirurgischen Sondierungsvorrichtung zuzuführen.

4. Sterilisationsvorrichtung nach Anspruch 2 oder 3, wobei der Durchgang durch den gasdichten Adapter (28, 60) hindurch eine Dichtung umfasst, um sicherzustellen, dass der Adapter (28, 60) gasdicht ist, wenn das Sterilisationsinstrument in dem Durchgang positioniert wird.

5. Sterilisationsvorrichtung nach einem der Ansprüche 2 bis 4, wobei der gasdichte Adapter (60) einen Luer-Lock-Anschluss (63) zum Befestigen des gasdichten Adapters (28, 60) an der chirurgischen Sondierungsvorrichtung umfasst.

6. Sterilisationsvorrichtung nach einem der vorangegangenen Ansprüche, wobei die Sterilisationsvorrichtung (10, 20) ferner eine Schließvorrichtung (26) umfasst, die ausgelegt ist, um ein distales Ende des Kanals der chirurgischen Sondierungsvorrichtung abzudichten.

7. Sterilisationsvorrichtung nach Anspruch 6, wobei entweder:
die Gaszufuhr (16) mit der Schließvorrichtung (26) verbindbar ist, um das ionisierbare Gas in den Kanal der chirurgischen Sondierungsvorrichtung zuzuführen; oder
die Schließvorrichtung (26) ein Druckablassventil (27) umfasst.

8. Sterilisationsvorrichtung nach einem der vorangegangenen Ansprüche, wobei die Elektrodenanordnung eine erste Elektrode (42) und eine zweite Elektrode (44) umfasst, die angeordnet sind, um ein Volumen (48) zwischen diesen zu definieren, und wobei die erste Elektrode (42) und die zweite Elektrode (44) ausgelegt sind, um die HF- und/oder Mikrowellenenergie von der Übertragungsleitung zu empfangen, um ein elektrisches Feld in dem Volumen (48) zum Erzeugen eines Plasmas des ionisierbaren Gases zu erzeugen; und
wobei die Sondenspitze (40) einen Einlass umfasst, um zu ermöglichen, dass ionisierbares Gas aus dem Kanal der chirurgischen Sondierungsvorrichtung und in das Volumen (48), das zwischen der ersten Elektrode (42) und der zweiten Elektrode (44) definiert ist, strömt.

9. Sterilisationsvorrichtung nach Anspruch 8, wobei:
die Übertragungsleitung ein Koaxialkabel umfasst, das einen Innenleiter, einen Außenleiter und ein dielektrisches Material umfasst, das den Innenleiter von dem Außenleiter trennt,
die zweite Elektrode (44) eine Verlängerung des Außenleiters des Koaxialkabels über das dielektrische Material des Koaxialkabels hinaus umfasst und
die erste Elektrode (42) eine Verlängerung des Innenleiters des Koaxialkabels über das dielektrische Material des Koaxialkabels hinaus und entlang einer Mittelachse der zweiten Elektrode (44) umfasst, um das Volumen (48) zwischen der ersten Elektrode (42) und der zweiten Elektrode (44) zu definieren;
wobei die erste Elektrode (42) und die zweite Elektrode (44) ausgelegt sind, um die HF- und/oder Mikrowellenenergie von dem Koaxialkabel zu empfangen, um ein elektrisches Feld in dem Volumen (48) zwischen der ersten Elektrode (42) und der zweiten Elektrode (44) zum Zünden eines Plasmas in diesem zu erzeugen, und
wobei die Sondenspitze (40) einen Auslass zum Freisetzen des Plasmas aus dem Volumen (48) umfasst.

10. Sterilisationsvorrichtung nach Anspruch 9, wobei die zweite Elektrode (44) zumindest eine Öffnung umfasst, um den Einlass in die Sondenspitze (40) zu definieren, um zu ermöglichen, dass ionisierbares Gas aus dem Kanal der chirurgischen Sondierungsvorrichtung und in das Volumen (48) strömt.

11. Sterilisationsvorrichtung nach Anspruch 9 oder Anspruch 10, wobei die erste Elektrode (42) eine leitfähige Kappe (54) umfasst, die auf einem distalen Ende der Verlängerung des Innenleiters angebracht ist, wobei die leitfähige Kappe (54) von dem distalen Ende der zweiten Elektrode (44) beabstandet ist, um einen Spalt zwischen der Kappe und der zweiten Elektrode (44) zu definieren, um den Auslass zu definieren.

12. Sterilisationsvorrichtung nach Anspruch 11, wobei die leitfähige Kappe (54) zumindest eine Öffnung umfasst, um den Einlass in die Sondenspitze (40) zu definieren, um zu ermöglichen, dass ionisierbares Gas aus dem Kanal der chirurgischen Sondierungsvorrichtung und in das Volumen (48) strömt.

13. Sterilisationsvorrichtung nach einem der Ansprüche 9 bis 12, wobei die Sondenspitze (40) ferner ein Stück eines isolierenden dielektrischen Materials umfasst, das zwischen der ersten Elektrode (42) und der zweiten Elektrode (44) angeordnet ist.

14. Sterilisationsvorrichtung nach einem der vorangegangenen Ansprüche, wobei die längliche Sonde einen Durchmesser von 1 mm oder weniger aufweist.

15. Sterilisationsvorrichtung nach einem der vorangegangenen Ansprüche, wobei die Gaszufuhr (16) ausgelegt ist, um Gas mit einer variierbaren Strömungsrate in den Kanal der chirurgischen Sondierungsvorrichtung zuzuführen.

## Revendications

1. Appareil de stérilisation pour stériliser un canal d'un dispositif d'endoscopie chirurgicale, l'appareil comprenant :
une alimentation en gaz (16),
un instrument de stérilisation configuré pour être inséré à travers le canal d'un dispositif d'endoscopie chirurgicale, l'instrument de stérilisation comprenant :
une sonde allongée comprenant :
un ligne de transmission (32) pour transporter de l'énergie électromagnétique (EM) radiofréquence (RF) et/ou de l'énergie EM hyperfréquence, et
une pointe de sonde (40) connectée à l'extrémité distale de la ligne de transmission pour recevoir l'énergie EM RF et/ou hyperfréquence, la pointe de sonde (40) comprenant un ensemble d'électrodes configuré pour produire un champ électrique à partir de l'énergie EM RF et/ou hyperfréquence reçue afin de générer un plasma du gaz ionisable délivré au canal à partir de l'alimentation en gaz (16), **caractérisé en ce que** :
l'alimentation en gaz présente un conduit pour délivrer un gaz ionisable au canal du dispositif d'endoscopie chirurgicale, et **en ce que**
l'instrument de stérilisation est séparé de l'alimentation en gaz et du conduit, et **en ce que**
l'instrument de stérilisation est exempt d'un conduit pour transporter du gaz ionisable pour la génération d'un plasma par l'ensemble d'électrodes.

2. Appareil de stérilisation selon la revendication 1, l'appareil de stérilisation (10, 20) comprenant en outre un adaptateur étanche aux gaz (28, 60) configuré pour être connectable au dispositif d'endoscopie, l'adaptateur étanche aux gaz présentant un passage à travers celui-ci pour permettre à l'instrument de stérilisation d'être introduit dans le canal du dispositif d'endoscopie à travers l'adaptateur étanche aux gaz (28, 60).

3. Appareil de stérilisation selon la revendication 2, dans lequel :
l'adaptateur étanche aux gaz (28, 60) comprend une soupape de décharge (27) ; ou
l'alimentation en gaz (16) est configurée pour pouvoir être connectée à l'adaptateur (28, 60) afin de délivrer le gaz ionisable au canal du dispositif d'endoscopie chirurgicale.

4. Appareil de stérilisation selon la revendication 2 ou la revendication 3, dans lequel le passage à travers l'adaptateur étanche aux gaz (28, 60) comprend un joint d'étanchéité pour assurer que l'adaptateur (28, 60) soit étanche aux gaz lorsque l'instrument de stérilisation est positionné dans le passage.

5. Appareil de stérilisation selon l'une quelconque des revendications 2 à 4, dans lequel l'adaptateur étanche aux gaz (60) comprend un raccord à verrouillage luer (63) pour fixer l'adaptateur étanche aux gaz (28, 60) au dispositif de d'endoscopie chirurgicale.

6. Appareil de stérilisation selon l'une quelconque des revendications précédentes, l'appareil de stérilisation (10, 20) comprenant en outre un dispositif de fermeture (26) configuré pour sceller une extrémité distale du canal du dispositif de d'endoscopie chirurgicale.

7. Appareil de stérilisation selon la revendication 6, dans lequel :
l'alimentation en gaz (16) peut être connectée au dispositif de fermeture (26) pour délivrer le gaz ionisable au canal du dispositif d'endoscopie chirurgicale ; ou
le dispositif de fermeture (26) comprend une soupape de décharge (27).

8. Appareil de stérilisation selon l'une quelconque des revendications précédentes, dans lequel l'ensemble d'électrodes comprend une première électrode (42) et une seconde électrode (44) agencées pour définir un volume (48) entre celles-ci, et la première électrode (42) et la seconde électrode (44) sont configurées pour recevoir de l'énergie RF et/ou hyperfréquence à partir de la ligne de transmission afin d'établir un champ électrique dans le volume (48) pour produire un plasma du gaz ionisable ; et
dans lequel la pointe de sonde (40) comprend une entrée pour permettre au gaz ionisable de s'écouler à partir du canal du dispositif d'endoscopie chirurgicale et jusque dans le volume (48) défini entre la première électrode (42) et la seconde électrode (44).

9. Appareil de stérilisation selon la revendication 8, dans lequel
la ligne de transmission comprend un câble coaxial présentant un conducteur interne, un conducteur externe et un matériau diélectrique séparant le conducteur interne du conducteur externe,
la seconde électrode (44) comprend un prolongement du conducteur externe du câble coaxial au-delà du matériau diélectrique du câble coaxial, et
la première électrode (42) comprend un prolongement du conducteur interne du câble coaxial au-delà du matériau diélectrique du câble coaxial et le long d'un axe central de la seconde électrode (44) pour définir le volume (48) entre la première électrode (42) et la seconde électrode (44) ;
dans lequel la première électrode (42) et la seconde électrode (44) sont configurées pour recevoir l'énergie RF et/ou hyperfréquence à partir du câble coaxial afin d'établir un champ électrique dans le volume (48) entre la première électrode (42) et la seconde électrode (44) pour amorcer un plasma en son sein, et
dans lequel la pointe de sonde (40) inclut une sortie pour libérer du plasma à partir du volume (48).

10. Appareil de stérilisation selon la revendication 9, dans lequel la seconde électrode (44) comprend au moins une ouverture pour définir l'entrée dans la pointe de sonde (40) afin de permettre au gaz ionisable de s'écouler à partir du canal du dispositif d'endoscopie chirurgicale et jusque dans le volume (48).

11. Appareil de stérilisation selon la revendication 9 ou la revendication 10, dans lequel la première électrode (42) comprend un capuchon conducteur (54) monté sur une extrémité distale du prolongement du conducteur interne, dans lequel le capuchon conducteur (54) est espacé de l'extrémité distale de la seconde électrode (44) pour définir un espace entre le capuchon et la seconde électrode (44) afin de définir la sortie.

12. Appareil de stérilisation selon la revendication 11, dans lequel le capuchon conducteur (54) comprend au moins une ouverture pour définir l'entrée dans la pointe de sonde (40) afin de permettre au gaz ionisable de s'écouler à partir du canal du dispositif d'endoscopie chirurgicale et jusque dans le volume (48).

13. Appareil de stérilisation selon l'une quelconque des revendications 9 à 12, dans lequel la pointe de sonde (40) comprend en outre une pièce de matériau diélectrique isolant positionnée entre la première électrode (42) et la seconde électrode (44).

14. Appareil de stérilisation selon l'une quelconque des revendications précédentes, dans lequel la sonde allongée présente une diamètre de 1 mm ou moins.

15. Appareil de stérilisation selon l'une quelconque des revendications précédentes, dans lequel l'alimentation en gaz (16) est configurée pour fournir du gaz au canal du dispositif de détermination d'endoscopie chirurgicale à un débit variable.
